# EUROPÄISCHE PATENTSCHRIFT

(11) **EP 2 072 068 B1**
(45) Veröffentlichungstag und Bekanntmachung des Hinweises auf die Patenterteilung: **29.03.2017**
(21) Anmeldenummer: 08169550.4
(22) Anmeldetag: 20.11.2008
(51) Int. Cl.: A61L 31/02, A61L 31/08, A61L 31/14

(54) **IMPLANTAT MIT EINEM GRUNDKÖRPER AUS EINER BIOKORRODIERBAREN LEGIERUNG**
IMPLANT WITH A BASE BODY OF A BIOCORRODIBLE ALLOY
IMPLANT COMPOSÉ D'UN ALLIAGE BIOCORRODABLE

(30) Priorität: 20.12.2007 DE 102007061647
(43) Veröffentlichungstag der Anmeldung: 24.06.2009
(73) Patentinhaber: Biotronik VI Patent AG, 6341 Baar (CH)
(72) Erfinder: Becher, Bärbel, 18057, Rostock (DE); Momma, Carsten, 18055, Rostock (DE); Lootz, Daniel, 18119, Rostock (DE); Quade, Antje, 17094, Rowa (DE); Ohl, Andreas, 17440, Hohendorf (DE); Schröder, Karsten, 17489, Greifswald (DE)
(74) Vertreter: Galander, Marcus

(56) Entgegenhaltungen:
- WO-A1-87/01040
- WO-A1-89/11836
- DE-A1-102005 018 356
- US-A- 6 156 435
- US-A1- 2007 009 565
- US-A1- 2007 172 666
- VERWEIRE I ET AL: "Evaluation of fluorinated polymers as coronary stent coating", JOURNAL OF MATERIALS SCIENCE: MATERIALS IN MEDICINE, KLUWER ACADEMIC PUBLISHERS, BO, Bd. 11, Nr. 4, 1. April 2000 (2000-04-01), Seiten 207-212, XP019211941, ISSN: 1573-4838, DOI: 10.1023/A:1008908007424

## Beschreibung

Die Erfindung betrifft ein Implantat mit einem Grundkörper, der ganz oder in Teilen aus einer biokorrodierbaren Legierung der Elemente Magnesium, Eisen, Zink, Molybdän und Wolfram besteht. Ein weiterer Aspekt der Erfindung liegt in der Bereitstellung eines Verfahrens zur Herstellung eines solchen Stents.

### Technologischer Hintergrund und Stand der Technik

Implantate haben in vielfältiger Ausführungsform Anwendung in der modernen Medizintechnik gefunden. Sie dienen unter anderem der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper jedes Implantats, insbesondere von Stents, besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantats an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden. Für die Zwecke der vorliegenden Erfindung sind lediglich degradierbare/resorbierbare, metallische Implantatwerkstoffe von Interesse, die nachfolgend als biokorrodierbare metallische Werkstoffe bezeichnet werden.

Der Einsatz biokorrodierbarer metallischer Werkstoffe bietet sich insbesondere schon deshalb an, da zumeist nur ein zeitweiliger Verbleib des Implantats im Körper zur Erfüllung des medizinischen Zweckes erforderlich ist. Implantate aus permanenten Werkstoffen, also Werkstoffen, die im Körper nicht abgebaut werden, sind gegebenenfalls wieder zu entfernen, da es mittel- und langfristig auch bei hoher Biokompatibilität zu Abstoßungsreaktionen des Körpers kommen kann.

Ein Ansatz zur Vermeidung eines weiteren chirurgischen Eingriffs besteht demnach darin, das Implantat ganz oder in Teilen aus einem biokorrodierbaren metallischen Werkstoff zu formen. Unter Biokorrosion werden mikrobielle Vorgänge oder schlicht durch die Anwesenheit von Körpermedien bedingte Prozesse verstanden, die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert. Die Abbauprodukte haben, wie beispielsweise beim Magnesium, im günstigsten Fall sogar eine positive therapeutische Wirkung auf das umgebende Gewebe. Geringe Mengen nicht resorbierbarer Legierungsbestandteile sind - sofern sie nicht toxische sind -tolerierbar.

Bekannte biokorrodierbare metallische Werkstoffe umfassen Reineisen und biokorrodierbare Legierungen der Hauptelemente Magnesium, Eisen, Zink, Molybdän und Wolfram. In DE 197 31 021 A1 wird unter anderem vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines Stent, eignet. Ungeachtet der erreichten Fortschritte auf dem Gebiet biokorrodierbarer Metalllegierungen sind die bisher bekannten Legierungen aufgrund ihres Korrosionsverhaltens nur beschränkt einsatzfähig. Insbesondere limitiert die relativ rasche Biokorrosion der Magnesiumlegierungen deren Einsatzgebiet.

Herkömmliche technische Einsatzgebiete von Formkörpern aus metallischen Werkstoffen, insbesondere Magnesiumlegierungen, außerhalb der Medizintechnik erfordern in der Regel eine weitgehende Unterbindung korrosiver Prozesse. Dementsprechend ist die Zielstellung der meisten technischen Verfahren zur Verbesserung des Korrosionsverhaltens eine vollständige Inhibierung korrosiver Prozesse. Dagegen sollte die Zielstellung zur Verbesserung des Korrosionsverhaltens der hier vorliegenden biokorrodierbaren metallischen Werkstoffe nicht in der vollständigen Unterbindung, sondern nur in der Hemmung korrosiver Prozesse liegen. Schon aus diesem Grunde eignen sich die meisten bekannten Maßnahmen zur Verbesserung des Korrosionsschutzes nicht. Ferner müssen für einen medizintechnischen Einsatz auch toxikologische Aspekte berücksichtigt werden. Des Weiteren sind korrosive Prozesse stark von dem Medium abhängig, in dem sie ablaufen, und daher dürfte eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich sein.

Ein Ansatzpunkt bekannter technischer Verfahren zur Verbesserung des Korrosionsverhaltens (im Sinne einer Verstärkung des Korrosionsschutzes) sieht vor, eine korrosionsschützende Schicht auf dem aus dem metallischen Werkstoff bestehenden Formkörper zu erzeugen. Bekannte Verfahren zur Erzeugung einer korrosionsschützenden Schicht wurden unter dem Gesichtspunkt eines technischen Einsatzes des beschichteten Formkörpers - jedoch nicht medizintechnischen Einsatzes in biokorrodierbaren Implantaten in physiologischer Umgebung - entwickelt und optimiert. Diese bekannten Verfahren umfassen beispielsweise: das Aufbringen von Polymeren oder anorganischen Deckschichten, das Erzeugen einer Emaille, die chemische Konversion der Oberfläche, Heißgasoxidation, Anodisieren, Plasmaspritzen, Laserstrahl-Umschmelzen, PVD-Verfahren, Ionenimplantation oder Lackieren.

EP 0 993 308 B1 beschreibt einen permanenten Stent, der mittels eines PVD-Verfahrens mit einem Trägerpolymer beschichtet wird, an das Perfluoralkylketten gebunden sind. Aus EP 0 560 849 B1 ist ein Implantat mit einer fluorierten, polymeren Oberfläche bekannt, die durch das Eintauchen des Implantats in eine Lösung und anschließende Trocknung des Implantats erzeugt wird. US 5,246,451 offenbart ein Plasmabeschichtungsverfahren für permanente vaskuläre Prothesen, bei dem eine polymere, fluorhaltige Schicht durch eine Plasmabehandlung erzeugt werden kann. Diese Polymerschicht wird anschließend - wiederum unter Einsatz eines Plasmas - funktionalisiert.

US 6 156 435 offenbart ein Verfahren zur Herausbildung eines dünnen Polymerfilm aus Fluorkohlenstoff auf der Oberfläche einer Struktur.

US 2007/0172666 offenbart ein Plasmadepositionsverfahren zur Erzeugung eines hydrophoben fluorierten dünnen Films auf verschiedenen Oberflächen.

US 2007/0009565 offenbart ein Verfahren zur Funktionalisierung eines Polymers zur Herausbildung einer Beschichtung.

DE 102005018356 offenbart ein resorbierbares Implantat, welches vorrangig aus Zink, Calcium und/oder Magnesium besteht und eine biologisch abbaubare Beschichtung aufweisen kann.

Der vorliegenden Erfindung liegt die Aufgabe zugrunde, eine verbesserte oder zumindest alternative Beschichtung für ein Implantat aus einem biokorrodierbaren metallischen Werkstoff bereitzustellen, die eine temporäre Inhibition, aber nicht vollständige Unterbindung der Korrosion des Werkstoffs in physiologischer Umgebung bewirkt.

### Zusammenfassung der Erfindung

Die Erfindung geht aus von einem Implantat mit einem Grundkörper, der ganz aus einem biokorrodierbaren metallischen Werkstoff besteht. Das Implantat zeichnet sich dadurch aus, dass der aus biokorrodierbaren metallischen Werkstoff bestehende Gründkörper vollständig oder teilweise mit einer Beschichtung aus einer vernetzten CFₓ-Schicht mit einem F/C-Verhältnis im Bereich von 0.5 bis 1.5 bedeckt sind.

Das F/C-Verhältnis gibt neben der Oberflächenzusammensetzung auch Auskunft über die Vernetzungsanteile bzw. Vernetzungsgrad der aufgebrachten Schicht. Ein geringes F/C-Verhältnis wird durch eine stark vernetzte F/C-haltige Schicht verursacht. Ein hohes F/C-Verhältnis wie das von kommerziellem PTFE (F/C=2) deutet auf eine kettenförmige, sehr wenig vernetzte F/C-haltige Schicht. Vorzugsweise sind die aus dem biokorrodierbaren metallischen Werkstoff bestehenden Teile des Grundkörpers vollständig mit der CFₓ-Schicht bedeckt. Über das F/C-Verhältnis kann man das Korrosionsverhalten steuern. Bei gleicher Schichtdicke führt eine Erhöhung des F/C-Verhältnisses zu einer Verminderung der Korrosionsrate.

Es hat sich gezeigt, dass die Aufbringung einer Beschichtung der genannten Zusammensetzung nicht zur Ausbildung einer permanent vollständig oder weitgehend die Korrosion in physiologischer Umgebung inhibierenden Schutzschicht führt. Mit anderen Worten, in physiologischer Umgebung erfolgt dennoch eine Korrosion des Implantats, jedoch mit deutlich verzögerter Geschwindigkeit.

Nach einer bevorzugten Ausführungsform besitzt die CFₓ-Schicht eine Schichtdicke im Bereich von 1 nm bis 10 µm. Sofern das Implantat ein Stent ist, liegt eine Schichtdicke der CFₓ-Schicht vorzugsweise im Bereich von 1 nm bis 2 µm, insbesondere 50 nm bis 100 nm. Bei Schichtdicken unterhalb der angegebenen Untergrenze ist eine homogene Bedeckung der zu beschichtenden Bereiche des Grundkörpers nicht mehr sichergestellt, so dass eine reproduzierbare Einstellung eines gewünschten Korrosionsverhaltens erschwert ist. Oberhalb der genannten Grenze für die Schichtdicke können Eigenspannungen innerhalb der Schicht auftreten, die zu Inhomogenitäten führen, was wiederum eine reproduzierbare Einstellung des gewünschten Korrosionsverhaltens bedingen kann. Es versteht sich, dass mit steigender Schichtdicke die korrosionshemmende Wirkung der CFₓ-Schicht zunimmt. Zur Erzielung eines vorgebbaren Korrosionsverhaltens kann der Fachmann wie folgt vorgehen:
Es werden Probenkörper aus dem biokorrodierbaren metallischen Werkstoff hergestellt und diese werden nach dem erfindungsgemäßen Verfahren beschichtet bis sich eine jeweils vorgebbare Schichtdicke der CFₓ-Schicht einstellt. Auf diese Weise können beispielsweise fünf Probenkörper mit unterschiedlich definierter Schichtdicke hergestellt werden, deren Korrosionsverhalten anschließend quantifiziert wird (zum Beispiel durch Bestimmung der Korrosionsrate) und die eine qualitative Voraussage des Zusammenhangs zwischen Schichtdicke und Korrosionsverhalten erlauben. Die erhaltenen Daten für das Korrosionsverhalten werden mit dem gewünschten Korrosionsverhalten verglichen. Zeigt der Vergleich noch signifikante Abweichungen zu jedem der von den Probenkörpern erhaltenen Werte, wird ausgehend von dem am nächsten liegenden Wert die Schichtdicke in weiteren Probenkörpern variiert. Letztendlich kann der Fachmann durch routinemäßiges Abarbeiten dieser Optimierungsprozedur eine Schichtdicke für das gewünschte Korrosionsverhalten ermitteln.

Vorzugsweise ist der biokorrodierbare metallische Werkstoff eine biokorrodierbare Legierung ausgewählt aus der Gruppe der Elemente Magnesium, Eisen, Zink, Molybdän und Wolfram; insbesondere ist der Werkstoff eine biokorrodierbare Magnesiumlegierung. Unter Legierung wird vorliegend ein metallisches Gefüge verstanden, deren Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Besonders bevorzugt ist eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt. Diese Magnesiumlegierung bestätigte bereits in klinischen Versuchen ihre besondere Eignung, d. h. zeigt eine hohe Biokompatibilität, günstige Verarbeitungseigenschaften, gute mechanische Kennwerte. Durch in vivo Studien konnte gezeigt werden, dass die Magnesiumlegierung abgebaut beziehungsweise durch körpereigene Bestandteile ersetzt wird. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Bevorzugt sind weiterhin Magnesium-legierungen die bis zu 6 Gew.% Zink enthalten. Besonders bevorzugt ist weiterhin eine Magnesiumlegierung der Zusammensetzung Yttrium 0,5 - 10 Gew.%, Zink 0,5 - 6 Gew.%, Calcium 0,05 - 1 Gew.%, Mangan 0 - 0,5 Gew.%, Silber 0 - 1 Gew.%, Cer 0 - 1 Gew.% sowie Zirkonium 0 - 1 Gew.% oder Silizium 0 - 0,4 Gew.%, wobei sich die Angaben auf Gew.% an der Legierung beziehen und Magnesium sowie herstellungsbedingte Verunreinigungen den auf 100 Gew.% verbleibenden Restanteil an der Legierung einnehmen.

Die Legierungen der Elemente Magnesium, Eisen, Zink, Molybdän oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und mechanischer Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-OberflächenVerhältnis, Konzentrationsunterschied sowie Strömungsgeschwindigkeit beeinflusst.

An der Phasengrenzfläche zwischen Werkstoff und Medium laufen Redox-Reaktionen ab. Für eine schützende bzw. hemmende Wirkung müssen vorhandene Schutzschichten und/oder die Produkte der Redox-Reaktionen eine gegen das Korrosionsmedium ausreichend dichte Struktur ausbilden, eine bezogen auf die Umgebung erhöhte thermodynamische Stabilität aufweisen und im Korrosionsmedium wenig löslich oder unlöslich sein. In der Phasengrenzfläche, genauer in einer sich in diesem Bereich ausbildenden Doppelschicht, laufen Ad- und Desorptionsprozesse ab. Die Vorgänge in der Doppelschicht sind geprägt von den dort ablaufenden kathodischen, anodischen und chemischen Teilprozessen. Fremdstoffablagerungen, Verunreinigungen und Korrosionsprodukte beeinflussen den Korrosionsprozess. Die Vorgänge bei der Korrosion sind demnach hoch komplex und lassen sich gerade im Zusammenhang mit einem physiologischen Korrosionsmedium, also Blut oder künstlichem Plasma, nicht oder nur im geringen Umfang voraussagen, da Vergleichsdaten fehlen. Schon aus diesem Grunde ist das Auffinden einer korrosionshemmenden Beschichtung, d. h. einer Beschichtung, die nur zur temporären Herabsetzung der Korrosionsrate eines metallischen Werkstoffs der weiter oben genannten Zusammensetzung in physiologischer Umgebung dient, eine außerhalb der Routine eines Fachmanns liegende Maßnahme.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird eine im Sinne der Erfindung modifizierte Oberfläche zur Herabsetzung der Korrosionsrate führen. Im Fall von Koronarstents sollte vorzugsweise die mechanische Integrität der Struktur über ein Zeitraum von drei Monaten oder mehr nach Implantation aufrechterhalten werden.

Implantate im Sinne der Erfindung sind über ein chirurgisches Verfahren oder minimalinvasives Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes, beispielsweise Stents, und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten.

Vorzugsweise ist das Implantat ein Stent. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird. Bei Stents bestehen besondere Anforderungen an die korrosionshemmende Schicht: Die mechanische Belastung des Materials während der Expansion des Implantats hat Einfluss auf den Verlauf des Korrosionsprozesses und es ist davon auszugehen, dass die Spannungsrisskorrosion in den belasteten Bereichen verstärkt wird. Eine korrosionshemmende Schicht sollte diesen Umstand berücksichtigen. Weiterhin könnte eine harte korrosionshemmende Schicht während der Expansion des Stents abplatzen und eine Rissbildung in der Schicht bei Expansion des Implantats dürfte unvermeidbar sein. Schließlich sind die Dimensionen der filigranen metallischen Struktur zu beachten und es sollte nach Möglichkeit nur eine dünne, aber auch gleichmäßige korrosionshemmende Schicht erzeugt werden. Es hat sich nun gezeigt, dass das Aufbringen der erfindungsgemäßen Beschichtung ganz oder zumindest weitgehend diesen Anforderungen genügt.

Ein weiterer Aspekt der Erfindung liegt in der Bereitstellung eines Verfahrens zur Herstellung eines Implantats mit einem Grundkörper, der ganz aus einem biokorrodierbaren metallischen Werkstoff besteht, wobei der aus biokorrodierbaren metallischen Werkstoff bestehende Grünkörper vollständig oder teilweise mit einer Beschichtung aus einer vernetzten CFₓ-Schicht mit einem F/C-Verhältnis im Bereich von 0.5 bis 1.5 bedeckt ist. Das erfindungsgemäße Verfahren umfasst die Schritte:
(i) Bereitstellen einer Plasmabeschichtungsanlage sowie eines Implantatrohlings mit einem Grundkörper, der ganz aus dem biokorrodierbaren metallischen Werkstoff besteht; und
(ii) Beschichten der Rohlingsoberfläche in der Plasmabeschichtungs-anlage durch Plasmabehandlung in Gegenwart einer oder mehrerer Verbindungen ausgewählt aus der Gruppe umfassend Fluoralkane der Formel CₙF₂ₙ₊₂, Fluoralkene der Formel CₙF₂ₙ und Fluoralkine der Formel CₙF₂ₙ₋₂ sowie zyklische Fluorkohlenstoffverbindungen mit 3 bis 10 Kohlenstoffatomen, wobei n = 2 bis 10 ist, und unter den folgenden Bedingungen während der Plasmabehandlung:
   - einem Druck im Bereich von 0,01 bis 10 mbar,
   - einer Flussrate im Bereich von 1 bis 100 sccm und
   - einer eingekoppelten Leistung im Bereich von 300 bis 1000 W im Falle eines Mikrowellenplasmas oder einer eingekoppelten Leistung im Bereich von 10 bis 500 W im Falle eines Radiofrequenzplasmas.

Dem erfindungsgemäßen Verfahren liegt die Erkenntnis zugrunde, dass die Beschichtung von biokorrodierbaren metallischen Werkstoffen für Implantate sich besonders effektiv mit Hilfe eines Plasmaverfahrens durchführen lässt. Weiterhin ist die Wahl des Reaktivgases für die Plasmabeschichtung als auch der Betriebsparameter während der Plasmabehandlung ein wesentliches Element des erfindungsgemäßen Verfahrens, um zur gewünschten CFₓ-Schicht zu gelangen.

Für die Behandlung mit Plasma kann eine herkömmliche Plasmabeschichtungsanlage Einsatz finden. Das zu beschichtende Implantat sollte sich außerhalb der aktiven Zone im sogenannten "afterglow" des Plasmas befinden. Arbeiten im "afterglow" hat den Vorteil, dass keine elektrischen Felder auf die zu beschichtenden Proben (beispielsweise ein metallischer Stent) eingekoppelt werden können, die den Prozess durch unzulässige Überhitzungsgefahr stören könnten.

Vorzugsweise liegt die eingekoppelte Leistung im Falle einer Anregung des Plasmas durch Mikrowellen im Bereich von 500 bis 900 W.

Eine weiter bevorzugte Verfahrensvariante sieht vor, dass der herrschende Druck sowohl im Fall des Radiofrequenzplasmas als auch des Mikrowellenplasmas im Bereich von 0,1 mbar bis 5 mbar liegt.

Weiterhin ist bevorzugt wenn eine Kettenlänge n der eingesetzten Fluoralkanen, Fluoralkenen und Fluoralkinen Fluorcycloalkanen im Bereich von n = 3 bis n = 6 liegt. Zyklische Fluorkohlenstoffverbindungen mit 3 bis 10 Kohlenstoffatomen im Sinne der Erfindung umfassen alle Ringverbindungen mit einem ringförmigen Kohlenstoffgrundgerüst aus 3 bis 10 Kohlenstoffatomen, dessen freie Valenzen an den einzelnen Kohlenstoffatomen durch Fluor abgedeckt sind. Die zyklischen Fluorkohlenstoffverbindungen können C-C-Doppelbindungen und C-C-Dreifachbindungen enthalten und gegebenenfalls ein aromatisches System bilden. Besonders bevorzugt ist Hexafluorbenzol.

Schließlich ist bevorzugt, wenn die Flussrate, die sich über das Trägergas realisieren lässt, im Bereich von 30 bis 60 sccm, insbesondere bei 50 sccm liegt.

Die Erfindung wird nachfolgend anhand eines Ausführungsbeispiels näher erläutert.

Ein Stent aus der kommerziell erwerbbaren Magnesium-Legierung WE43 (nach ASTM) mit einem Seltenerdmetallanteil von etwa 3 Gew.% ohne Yttrium und einem Yttriumanteil von etwa 4 Gew.% wird in einer Plasmabeschichtungsanlage der Firma Plasma-finish GmbH eingebracht. Die Lage des Stents in der Beschichtungsanlage wird so vorgegeben, dass er sich im "afterglow" des zu erzeugenden Plasmas befindet. Anschließend wird als Reaktivgas Hexafluorbenzol C₆F₆ mit einer Flussrate von 50 sccm zugeführt, wobei Argon als Trägergas agiert. Der Prozessdruck beträgt 0.5 mbar. Die eingekoppelte Plasmaleistung wird auf 800 W geregelt, wobei die Anregung des Plasmas durch Mikrowellen erfolgt. Die Beschichtungszeit beträgt 2 min.

Nach der Entnahme des Stents kann die erhaltene vernetzte CFₓ-Schicht mit oberflächensensitiven Methoden strukturell nachgewiesen werden. Ein F/C-Verhältnis beträgt 0,6. Die Schichtdicke beträgt ca. 150 nm.

## Patentansprüche

1. Implantat mit einem Grundkörper, der ganz aus einem biokorrodierbaren metallischen Werkstoff besteht, **dadurch gekennzeichnet, dass** der aus biokorrodierbaren metallischen Werkstoff bestehende Grundkörper vollständig oder teilweise mit einer Beschichtung aus einer vernetzten CFₓ-Schicht mit einem F/C-Verhältnis im Bereich von 0.5 bis 1.5 bedeckt ist

2. Implantat nach Anspruch 1, bei dem der biokorrodierbare metallische Werkstoff eine biokorrodierbare Legierung ausgewählt aus der Gruppe Magnesium, Eisen, Zink, Molybdän und Wolfram ist.

3. Implantat nach Anspruch 2, bei dem der biokorrodierbare metallische Werkstoff eine Magnesiumlegierung ist.

4. Implantat nach einem der vorhergehenden Ansprüche, bei dem das Implantat ein Stent ist.

5. Implantat nach einem der vorhergehenden Ansprüche, bei dem die CFₓ-Schicht eine Schichtdicke im Bereich von 1 nm bis 10 µm aufweist.

6. Implantat nach Anspruch 5, bei dem das Implantat ein Stent ist und die CFₓ-Schicht eine Schichtdicke im Bereich von 1 nm bis 2 µm aufweist.

7. Implantat nach Anspruch 6, bei dem die CFₓ-Schicht eine Schichtdicke im Bereich von 50 nm bis 100 nm aufweist.

8. Verfahren zur Herstellung eines Implantats mit einem Grundkörper, der ganz aus einem biokorrodierbaren metallischen Werkstoff besteht, wobei der aus biokorrodierbaren metallischen Werkstoff bestehende Grundkörper vollständig oder teilweise mit einer Beschichtung aus einer vernetzten CFₓ-Schicht mit einem F/C-Verhältnis im Bereich von 0.5 bis 1.5 bedeckt ist und das Verfahren die Schritte umfasst:
(i) Bereitstellen einer Plasmabeschichtungsanlage sowie eines Implantatrohlings mit einem Grundkörper, der ganz aus dem biokorrodierbaren metallischen Werkstoff besteht; und
(ii) Beschichten der Rohlingsoberfläche in der Plasmabeschichtungsanlage durch Plasmabehandlung in Gegenwart einer oder mehrerer Verbindungen ausgewählt aus der Gruppe umfassend Fluoralkane der Formel CₙF₂ₙ₊₂, Fluoralkene der Formel CₙF₂ₙ, Fluoralkine der Formel CₙF₂ₙ₋₂ und zyklische Fluorkohlenstoffverbindungen mit 3 bis 10 Kohlenstoffatomen, wobei n = 2 bis 10 ist, und unter den folgenden Bedingungen während der Plasmabehandlung:
- einem Druck im Bereich von 0,01 bis 10 mbar,
- einer Flussrate im Bereich von 1 bis 100 Ncm³/s (sccm) und
- einer eingekoppelten Leistung im Bereich von 300 bis 1000 W im Falle eines Mikrowellenplasmas oder einer eingekoppelten Leistung im Bereich von 10 bis 500 W im Falle eines Radiofrequenzplasmas.

9. Verfahren nach Anspruch 8, bei dem ein Druck im Schritt (ii) im Bereich von 0,1 bis 5 mbar liegt.

10. Verfahren nach Anspruch 8 oder 9, bei dem eine Flussrate im Schritt (ii) im Bereich von 30 bis 60 Ncm³/s (sccm) liegt.

11. Verfahren nach einem der Ansprüche 8 bis 10, bei dem im Falle eines Mikrowellenplasmas eine eingekoppelte Leistung im Schritt (ii) im Bereich von 500 bis 900 W liegt.

12. Verfahren nach einem der Ansprüche 8 bis 11, bei dem n = 3 bis 6 ist.

## Claims

1. An implant, comprising a main body consisting wholly of a biocorrodible metallic material, **characterised in that** the main body consisting of biocorrodible metallic material is entirely or partially covered with a coating formed of a crosslinked CFₓ layer having an F/C ratio in the range of 0.5 to 1.5.

2. The implant according to claim 1, in which the biocorrodible metallic material is a biocorrodible alloy selected from the group consisting of magnesium, iron, zinc, molybdenum and tungsten.

3. The implant according to claim 2, in which the biocorrodible metallic material is a magnesium alloy.

4. The implant according to any one of the preceding claims, in which the implant is a stent.

5. The implant according to any one of the preceding claims, in which the CFₓ layer has a layer thickness in the range of 1 nm to 10 µm.

6. The implant according to claim 5, in which the implant is a stent and the CFₓ layer has a layer thickness in the range of 1 nm to 2 µm.

7. The implant according to claim 6, in which the CFₓ layer has a layer thickness in the range of 50 nm to 100 nm.

8. A method for producing an implant comprising a main body consisting entirely of a biocorrodible metallic material, wherein the main body consisting of the biocorrodible metallic material are entirely or partially covered with a coating formed of a crosslinked CFₓ layer having an F/C ratio in the range of 0.5 to 1.5, the method comprising the steps of:
(i) providing a plasma coating system and an implant blank having a main body consisting entirely of the biocorrodible material; and
(ii) coating the surface of the blank in the plasma coating system by plasma treatment in the presence of one or more compounds selected from the group comprising fluoroalkanes of the formula CₙF₂ₙ₊₂, fluoroalkenes of the formula CₙF₂ₙ, fluoroalkynes of the formula CₙF₂ₙ₋₂, and cyclic fluorocarbon compounds with 3 to 10 carbon atoms, wherein n=2 to 10, and under the following conditions during the plasma treatment:
- a pressure is in the range of 0.01 to 10 mbar,
- a flow rate is in the range of 1 to 100 Ncm³/s (sccm), and
- a power input is in the range of 300 to 1000 W in the case of a microwave plasma or a power input is in the range of 10 to 500 W in the case of a radiofrequency plasma.

9. The method according to claim 8, in which a pressure in step (ii) is in the range of 0.1 to 5 mbar.

10. The method according to claim 8 or 9, in which a flow rate in step (ii) is in the range of 30 to 60 Ncm³/s (sccm).

11. The method according to any one of claims 8 to 10, in which said power input in step (ii) is in the range of 500 to 900 W in the case of a microwave plasma.

12. The method according any one of claims 8 to 11, in which n=3 to 6.

## Revendications

1. Implant avec un corps de base qui est totalement constitué d'un matériau métallique biocorrodable, **caractérisé en ce que** le corps de base constitué du matériau métallique biocorrodable est recouvert complètement ou partiellement avec un revêtement à base d'une couche de CFₓ réticulé avec un rapport F/C dans la plage de 0,5 à 1,5.

2. Implant selon la revendication 1, chez lequel le matériau métallique biocorrodable est un alliage biocorrodable choisi dans le groupe du magnésium, du fer, du zinc, du molybdène et du tungstène.

3. Implant selon la revendication 2, chez lequel le matériau métallique biocorrodable est un alliage de magnésium.

4. Implant selon l'une des revendications précédentes, où l'implant est un stent.

5. Implant selon l'une des revendications précédentes, chez lequel la couche de CFₓ présente une épaisseur de couche dans la plage de 1 nm à 10 µm.

6. Implant selon la revendication 5, où l'implant est un stent et la couche de CFₓ présente une épaisseur de couche dans la plage de 1 nm à 2 µm.

7. Implant selon la revendication 6, chez lequel la couche de CFₓ présente une épaisseur de couche dans la plage de 50 nm à 100 nm.

8. Procédé de fabrication d'un implant avec un corps de base qui est totalement constitué d'un matériau métallique biocorrodable, où le corps de base constitué du matériau métallique biocorrodable est complètement ou partiellement recouvert avec un revêtement à base d'une couche de CFₓ réticulé avec un rapport F/C dans la plage de 0,5 à 1,5, et le procédé comprend les étapes :
(i) de fourniture d'une installation de revêtement par plasma ainsi que d'une ébauche d'implant avec un corps de base, qui est totalement constitué d'un matériau métallique biocorrodable ; et
(ii) de revêtement de la surface d'ébauche dans une installation de revêtement par plasma par un traitement par plasma en présence d'un ou de plusieurs composés choisis dans le groupe comprenant des fluoro-alcanes selon la formule CₙF₂ₙ₊₂, des fluoro-alcènes selon la formule CₙF₂ₙ, des fluoro-alcynes selon la formule CₙF₂ₙ₋₂ ou des composés de fluorocarbures cycliques avec 3 à 10 atomes de carbone, où n est = 2 à 10, et dans les conditions suivantes pendant le traitement par plasma :
- une pression dans la plage de 0,01 à 10 mbar,
- un débit dans la plage de 1 à 100 Ncm³/s (sccm) et
- une puissance impliquée dans la plage de 300 à 1000 W dans le cas d'un plasma à micro-ondes ou d'une puissance impliquée dans la plage de 10 à 500 W dans le cas d'un plasma de radiofréquence.

9. Procédé selon la revendication 8, chez lequel une pression dans l'étape (ii) se situe dans la plage de 0,1 à 5 mbar.

10. Procédé selon la revendication 8 ou 9, chez lequel un débit dans l'étape (ii) se situe dans la plage de 30 à 60 Ncm³/s (sccm).

11. Procédé selon l'une des revendications 8 à 10, chez lequel, dans le cas d'un plasma à micro-ondes, une puissance impliquée dans l'étape (ii) se situe dans la plage de 500 à 900 W.

12. Procédé selon l'une des revendications 8 à 11, chez lequel n est = 3 à 6.
